# EUROPEAN PATENT SPECIFICATION

(11) **EP 4 210 567 B1**
(45) Date of publication and mention of the grant of the patent: **15.04.2026**
(21) Application number: 21789938.4
(22) Date of filing: 14.09.2021
(51) Int. Cl.: A61B 5/0215, A61B 5/024, A61B 5/00

(54) **FLEXIBLE PRESSURE SENSOR WITH WIRELESS MONITORING CAPABILITY**
FLEXIBLER DRUCKSENSOR MIT DRAHTLOSER ÜBERWACHUNGSFÄHIGKEIT
CAPTEUR DE PRESSION FLEXIBLE À CAPACITÉ DE SURVEILLANCE SANS FIL

(30) Priority: 14.09.2020 US 202063077808 P; 20.07.2021 US 202163223653 P
(43) Date of publication of application: 19.07.2023
(73) Proprietor: CASE WESTERN RESERVE UNIVERSITY, Cleveland, Ohio 44106 (US); The Department of Veteran Affairs, Washington, DC 20420 (US)
(72) Inventor: MAJERUS, Steve J.A., Washington, DC 20420 (US); ZORMAN, Christian, Cleveland, Ohio 44106 (US); CHONG, Hao, Cleveland, Ohio 44106 (US); BASKIN, Jonathan, Cleveland, OH 44106 (US); TYLER, Dustin J., Cleveland, Ohio 44106 (US); PINAULT, Gilles, Washington, DC 20420 (US)
(74) Representative: Marks & Clerk LLP
(86) International application number: PCT/US2021/050282
(87) International publication number: WO 2022/056468

(56) References cited:
- US-A1- 2012 283 580
- US-A1- 2016 317 095
- US-A1- 2019 110 747

## Description

### Technical Field

The present disclosure relates to pressure sensing, more specifically, to a flexible pulsation sensor (FPS) device, including a FPS and a flexible circuit, that provides wireless monitoring capability.

### Background

Detecting blood flow and blood pressure within a conduit (e.g., a natural vessel or a vascular graft) can help physicians determine the health and safety of the conduit and/or the patient having the conduit. Hypertension (HTN) has been called the silent killer, causing consequences such as vascular disease and end-organ failure in multiple systems. Having a better, more exact, way to detect blood flow and blood pressure may reduce the effects of HTN. Moreover, over one million vascular grafts are implanted in the United States every year to help with many applications, including vascular bypass, vascular access for hemodialysis, etc. However, vascular grafts are vulnerable to failure (e.g., due to intimal hyperplasia where endothelial cell migration leads to reduced diameter in the graft lumen, blood clotting, reduced graft blood flow, and eventual graft occlusion). Detecting blood flow and/or blood pressure within the vascular grafts would help to detect the potential for failure before the failure happens. Testing of blood flow and/or blood pressure would also have utility for microvascular reconstruction where autogenous tissue is harvested from one part of the body with its feeding vessels intact with the intent of reconstructing a defect in another part of the body. The autograft is positioned in the defect and its arteries and veins are then anastomosed to recipient vessels in close proximity to the defect.
US 2016/317095 A1 relates to an implantable device including a body part and a piezoelectric part, where the body part is configured to grasp a pulsatile organic or inorganic tissue. The implantable device may comprise an electrical circuitry which may comprise a semiconductor chip or flexible polymer electronics, such as printed polymer electronics.
US 2019/110747 A1 relates to a sensor apparatus including at least one substrate layer of an elastically deformable material, the substrate layer extending longitudinally between spaced apart ends thereof. A conductive layer is attached to and extends longitudinally between the spaced apart ends of the at least one substrate layer. In an example, the conductive layer is formed of a piezoresistive elastomer, such as a conductive PDMS or other material. The sensor apparatus thus is sufficiently compliant to deform in a radial or circumferential direction corresponding to sensor strain (e.g., from about 0% to about 20% strain) in response to pulsatile blood flow through the graft that causes graft wall motion. Such deformation causes a change in electrical resistance in the conductive layer that can be measured over time.

### Summary of the Invention

The present invention provides an apparatus as defined in claim 1. Preferred embodiments of the invention are defined in the dependent claims.

### Summary of the Disclosure

Provided herein is a flexible pulsation sensor (FPS) device, including a FPS and a flexible circuit, that provides wireless monitoring capability and methods for using the FPS device. The FPS device can be placed around the outside of conduit (e.g., a vessel, a vascular graft, or the like) and flex with an increase/decrease in blood (or other fluid) pressure within the conduit and also may detect the rate of blood flow within the conduit. The FPS device is superior to previous sensors to detect a change in blood (or other fluid) pressure and/or blood flow because the FPS need not be within the lumen of the conduit.

In one aspect, the present disclosure includes an apparatus (a FPS device). The apparatus includes a FPS configured to wrap around a measurement target (e.g., a conduit, such as a vessel, a vascular graft, or the like) and a flexible circuit board. The flexible circuit board includes a sensor interface circuit on the flexible circuit board configured to collect data related to displacement of the FPS related to a pressure of and/or within the measurement target; and a wireless transmitter on the flexible circuit board configured to transmit the data related to the pressure of and/or within the measurement target wirelessly to an external device.

In another aspect, the present disclosure includes a method for using a FPS device. The method includes wrapping the FPS device around a measurement target; detecting, by the FPS device, a change of a pressure on and/or within the measurement target; and sending, by a wireless transmitter of the FPS device, data related to the change in the pressure to an external device.

### Brief Description of the Drawings

The foregoing and other features of the present disclosure will become apparent to those skilled in the art to which the present disclosure relates upon reading the following description with reference to the accompanying drawings, in which:
FIG. 1 is a diagram showing a flexible pulsation sensor (FPS) device;
FIGS. 2-5 are diagrams showing example flexible circuits that can be used by the FPS device of FIG. 1 to provide wireless monitoring capability;
FIG. 6 is a diagram showing an example of the FPS device of FIG. 1 wrapped around an conduit;
FIG. 7 is a process flow diagram of a method for using the FPS device of FIG. 1; and
FIGS. 8-14 are experimental figures used to demonstrate the feasibility of the FPS device of FIG. 1, specifically:

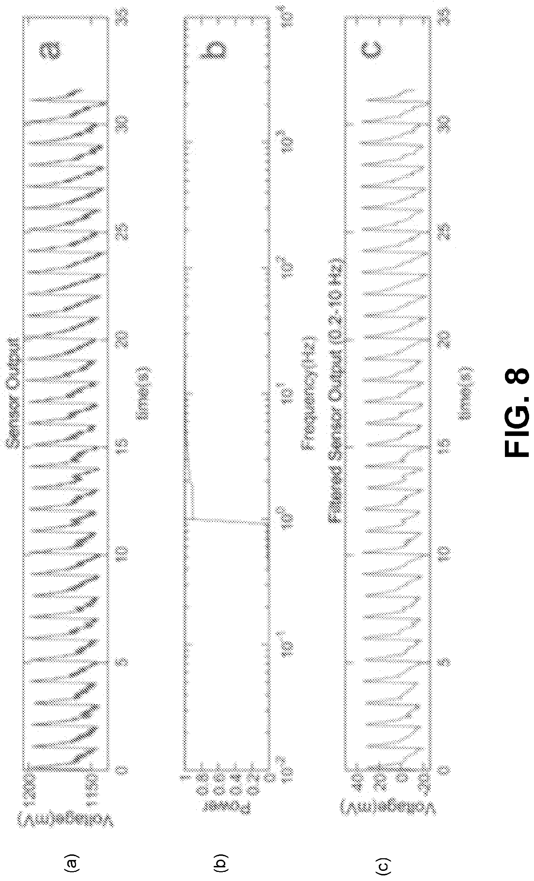

FIG. 8 shows plots used for determining parameters if a filter used by the flexible circuit;

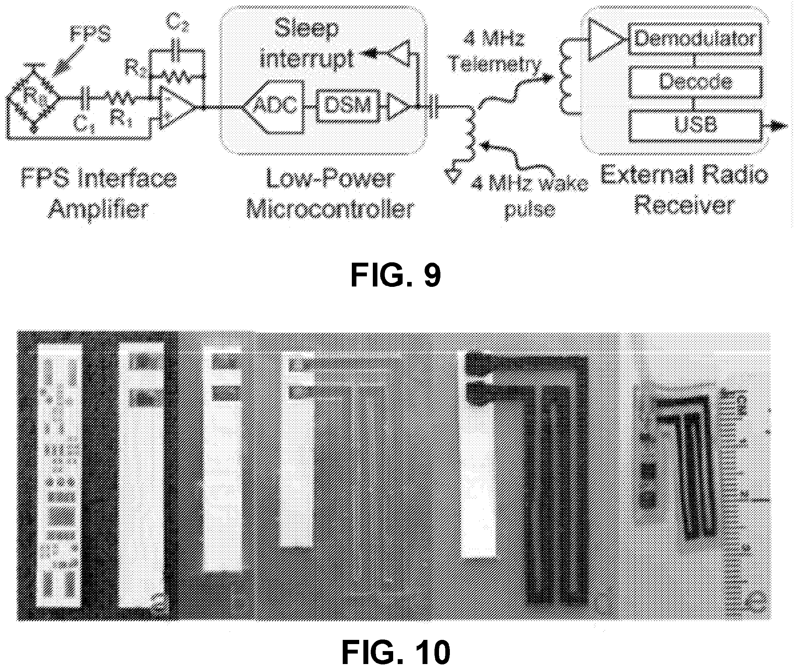

FIG. 9 is a schematic diagram of the wireless FPS device platform that uses a bridge amplifier to amplify and set the sensor signal bandwidth;
FIG. 10 includes photograms of the flexible circuit;

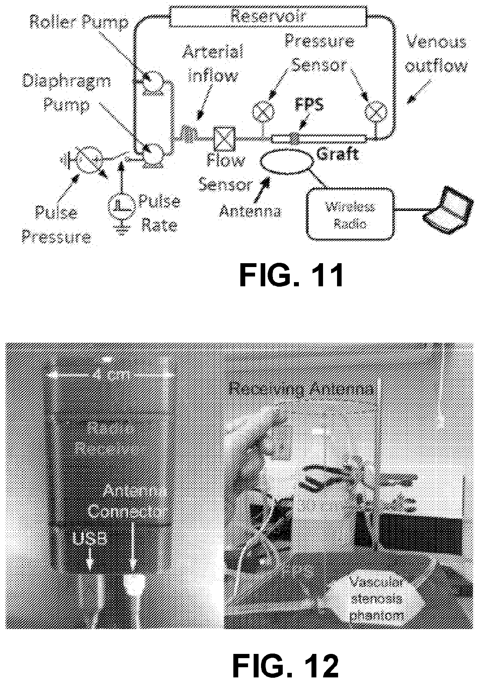

FIG. 11 is a schematic of the pulsatile graft flow circuit used for the in vitro test of the FPS device;
FIG. 12 includes photographs of the wireless radio for data reception and a wireless FPS device on a silicon phantom showing a transmission range of 30 cm;

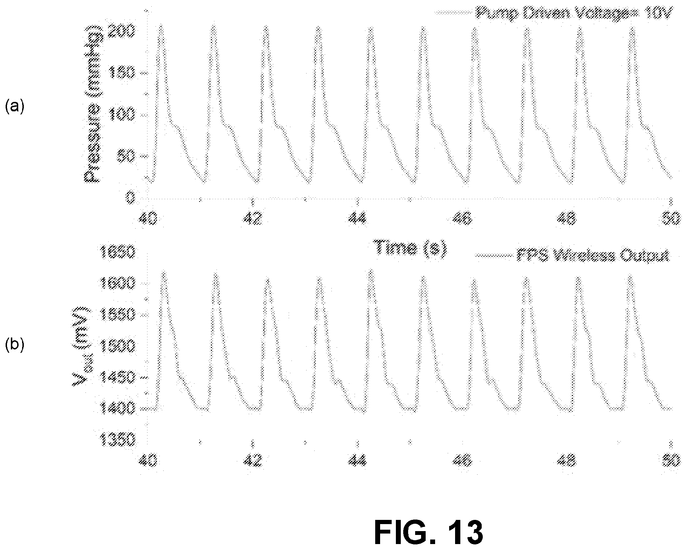

FIG. 13 includes plots of pressure data recorded by a commercial intraoperative blood pressure sensor (a) and pressure data recorded by the FPS device with an output signal transmitted wirelessly (b);

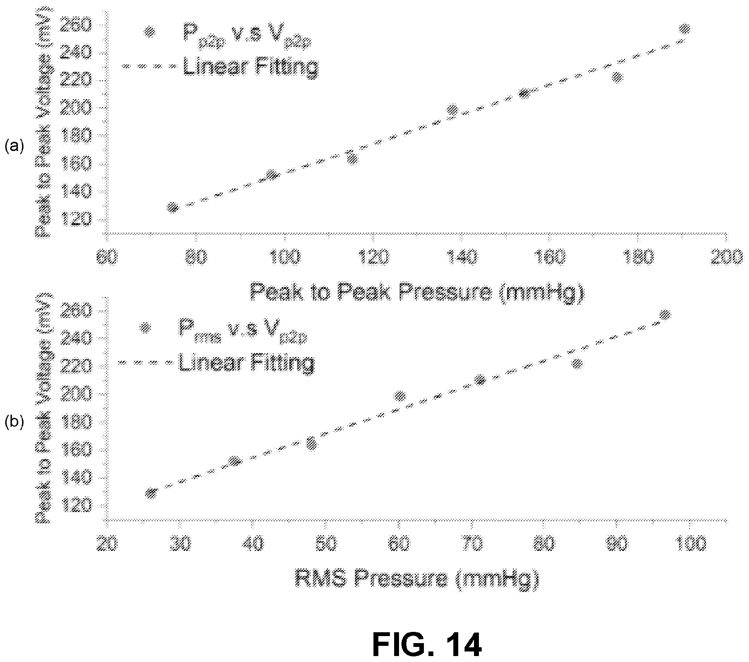

FIG. 14 includes plots showing comparisons between data from the FPS device vs. (a) diastolic-systolic pressure ranges and (b) RMS blood pressure under flows of 200-700 mL/min;

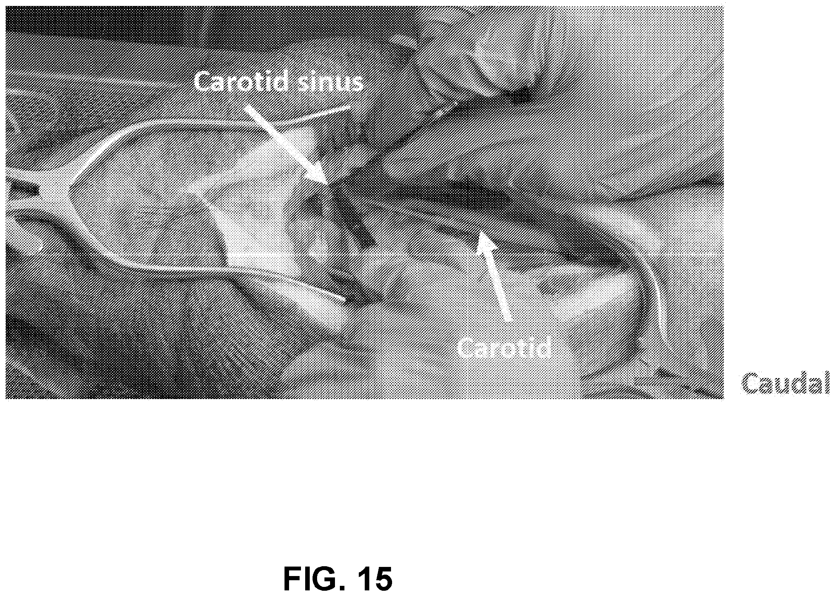

FIGS. 15-17 show photographs of a FPS being installed and tested in an adult Yucatan minipig cadaver;

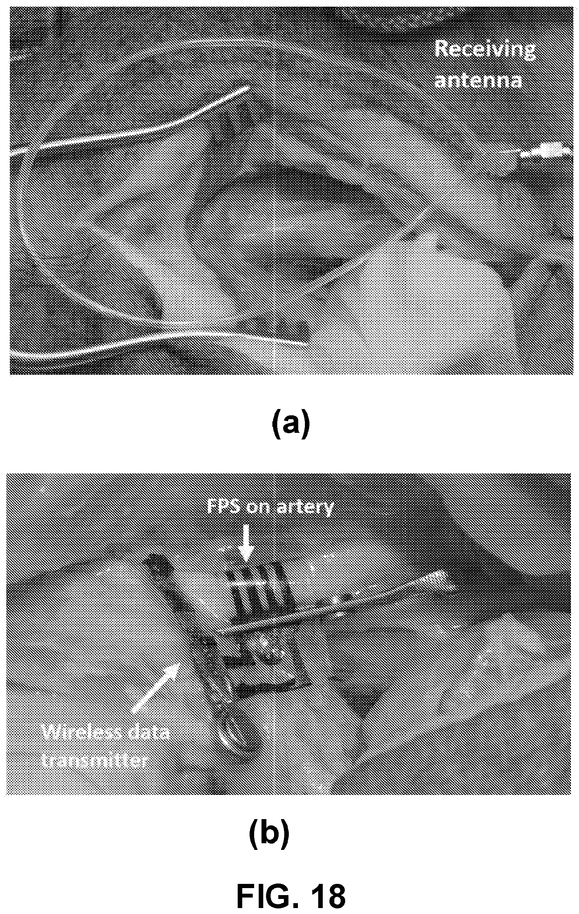

FIG. 18 shows a photograph of the implementation of wireless data collection from the FPS;

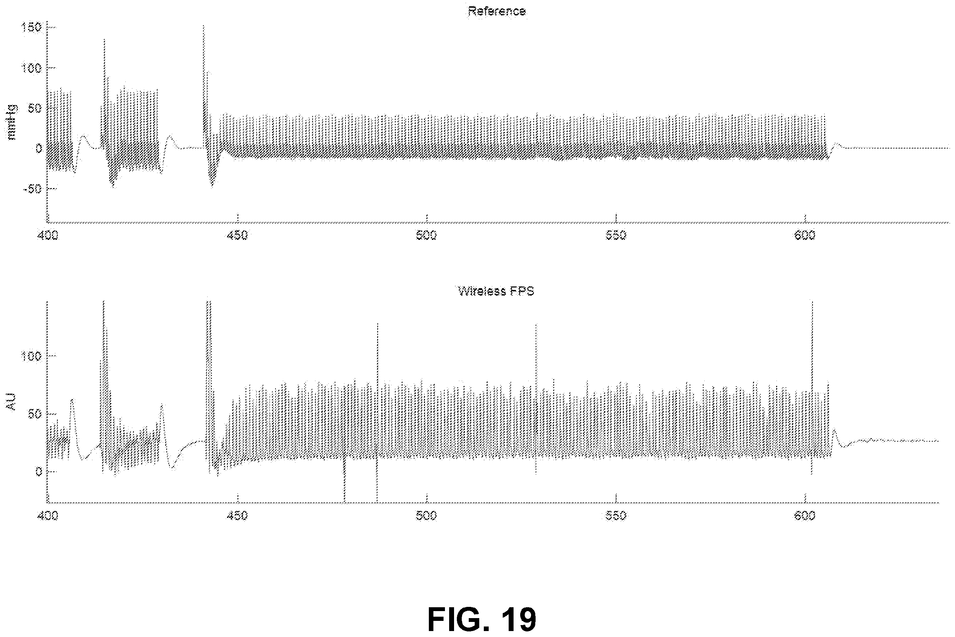

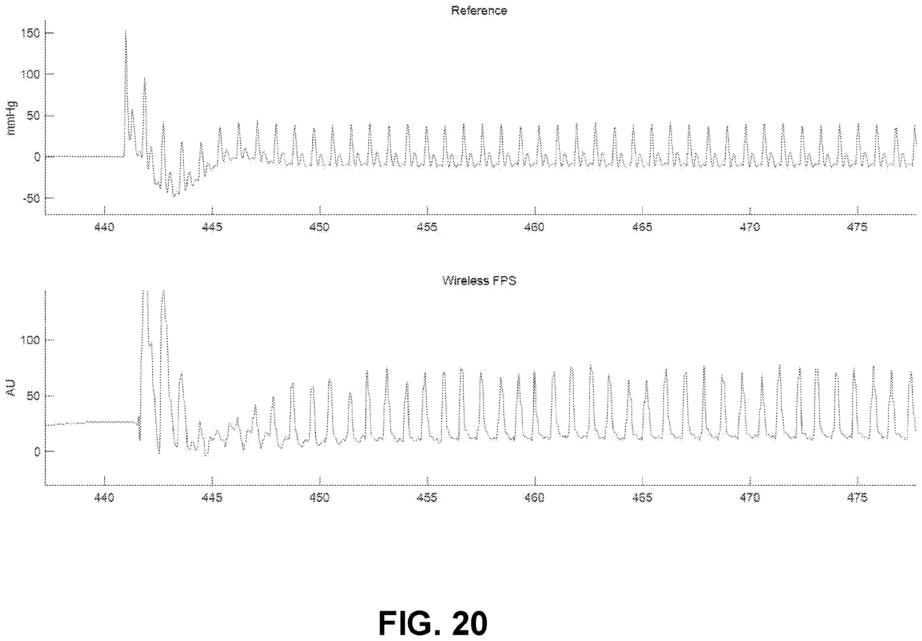

FIGS. 19 and 20 show example data recordings with correlation in flow and heart-rate measures by a reference sensor ("Reference") and the FPS ("Wireless FPS");

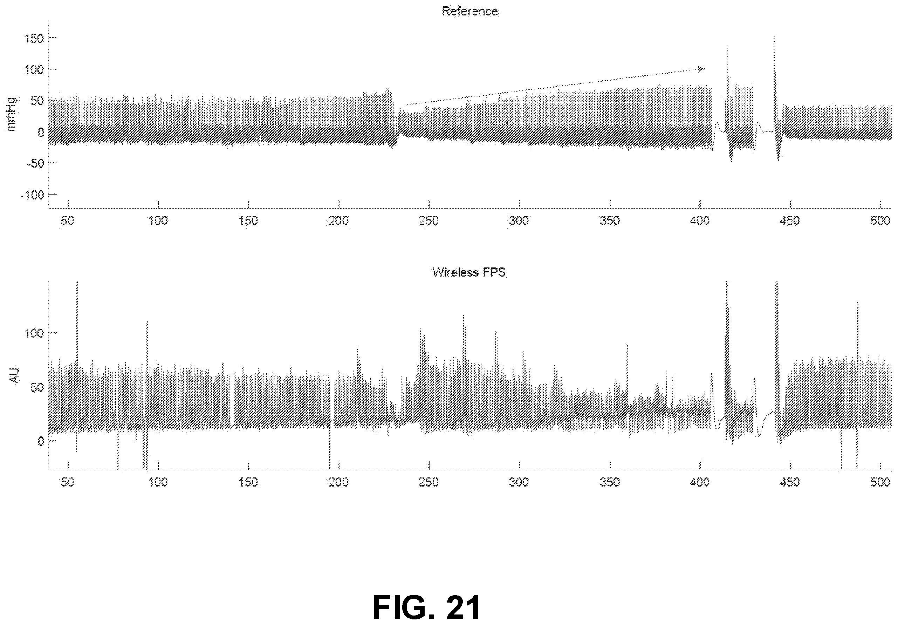

FIG. 21 shows an example of data recordings with increasing pump pressure and stagnation in the FPS signal; and

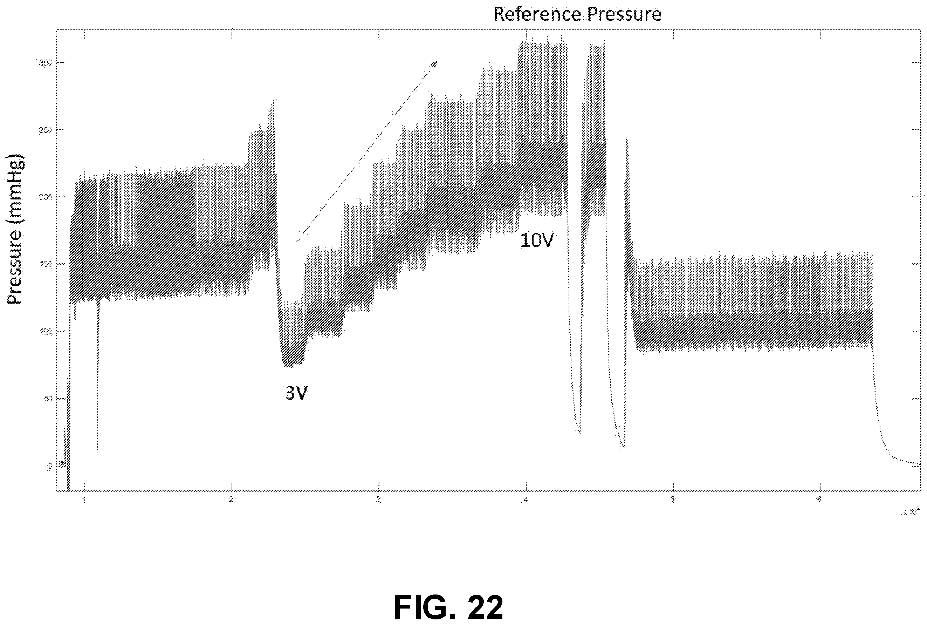

FIG. 22 shows a zoomed in portion of the plot of FIG. 21.

### Detailed Description

### I. Definitions

Unless otherwise defined, all technical terms used herein have the same meaning as commonly understood by one of ordinary skill in the art to which the present disclosure pertains.

As used herein, the singular forms "a," "an" and "the" can also include the plural forms, unless the context clearly indicates otherwise.

As used herein, the terms "comprises" and/or "comprising," can specify the presence of stated features, steps, operations, elements, and/or components, but do not preclude the presence or addition of one or more other features, steps, operations, elements, components, and/or groups.

As used herein, the term "and/or" can include any and all combinations of one or more of the associated listed items.

As used herein, the terms "first," "second," *etc.* should not limit the elements being described by these terms. These terms are only used to distinguish one element from another. Thus, a "first" element discussed below could also be termed a "second" element without departing from the teachings of the present disclosure. The sequence of operations (or acts/steps) is not limited to the order presented in the claims or figures unless specifically indicated otherwise.

As used herein, the term "flexible pulsation sensor (FPS)" can refer to a piezoresistive implantable strain sensor that can be wrapped around a measurement target. For example, the strain sensor can be made of a piezoresistive elastomer composite (e.g., with conductive particles, like conductive nanoparticles, within the elastomer).

As used herein, the term "piezoresistive" can refer to a change in electrical resistance that occurs when an external force is applied. The change affects a material's electrical resistivity.

As used herein, the term "strain sensor" can refer to a sensor whose resistance varies with applied force. The strain sensor can convert force, pressure, tension, weight, etc., into a change in electrical resistance, which can then be measured. The term "pressure sensor" can be used herein interchangeably with "strain sensor" with pressure being but one factor that can change the electrical resistance.

As used herein the term "measurement target" can refer to at least a portion of a conduit from which a parameter can be detected.

As used herein, the term "conduit" can refer to a channel configured to transport a fluid. In some instances, the conduit can have a generally cylindrical or tubular shape. Example conduits include a vascular graft, a vessel, or the like. Other cylindrical or tubular structures may include anything that can be similarly instrumented to measure strain or movement, for example, bones, intestines, or synthetic musculoskeletal implants, or the like for example, bones, intestines, or synthetic musculoskeletal implants, or the like

As used herein the term "vascular graft" can refer to a non-native tissue, a synthetic material, or an autograft, allograft, or xenograft, that is surgically implanted to reconnect blood vessels in order to redirect blood flow from one area to another.

As used herein the term "vessel" can refer to a vein or an artery that naturally forms part of the blood circulation system of the body. In some instances, a vessel may be one or more vessels that are a port of and feed a microvascular free flap that has been moved as an autograft from one part of the body to another.

As used here, the term "composite" can refer to something made up of various parts (e.g., an elastomer composite can be made of an elastomer and particles within the elastomer).

As used herein, the term "elastomer" can refer to a natural or synthetic polymer having elastic properties.

As used herein, the term "particle" can refer to a small piece of a larger material. For example, the particle can be a microparticle (on the micro-scale or less), a nanoparticle (on the nano-scale or less), or even smaller (e.g., a femto particle, on the femto-scale or less).

As used herein, the term "flexible pulsation sensor (FPS) device" can refer to something made or adapted to transmit data recorded by a FPS to an external device. The FPS device can include at least a wireless transmitter or transceiver to communicate with the external device.

As used herein, the term "flexible circuit" can refer to a thin insulating film having conductive circuit patterns affixed thereto (e.g., connecting to a filter, an amplifier, an interface, a wireless transmitter, an analog-to-digital convertor, etc.) and typically supplied with a thin polymer coating to protect the conductor parts.

As used herein, the term "biocompatible" can refer to something that is not harmful to living tissue.

### II. Overview

Described herein is a biocompatible flexible pulsation sensor (FPS) device that can be placed/wrapped around a measurement target to monitor a characteristic, such as blood pressure, blood flow, or the like. The measurement target can be a portion of a conduit, like a vessel, a vascular graft, or the like. In some instances, the FPS device can provide continuous monitoring of the characteristic. The FPS device integrates a FPS with a flexible wireless transmitter for real-time data recording and readout. The FPS device can have outstanding flexibility, being made of a conductive polymer sensing layer (e.g., constructed of a piezoresistive elastomer composite) attached to a flexible circuit (e.g., constructed on a flexible polyimide circuit board) with the wireless transmitter (e.g., provided by a low-power microcontroller). The FPS device can be fully encapsulated with polydimethylsiloxane (PDMS) to maintain flexibility and biocompatibility.

### III. System

Provided herein is an apparatus that provides a flexible sensor (e.g., a flexible pulsation sensor (FPS) 12) device with wireless monitoring capability. The apparatus can be referred to as a FPS device 10. In some instances, the FPS device 10 can transmit data wirelessly to an external device (not shown) for continuous monitoring of blood pressure, blood flow, or the like within a conduit (e.g., vessel, a graft, or the like). According to the present invention, the FPS device 10 is configured to wrap around a measurement target (e.g., a portion of a conduit). The size and geometry of the FPS device 10 can be adapted according to a given application and surface of the measurement target.

According to the present invention, the FPS device 10 includes a FPS 12 and a flexible circuit 14. The FPS device 10 can be biocompatible with a biocompatible coating (e.g., a layer of polydimethylsiloxane (PDMS)) over both the FPS 12 and the flexible circuit 14. However, any similar biocompatible coating may be used. It should be noted that the flexible circuit 14 can be in any location on or next to the FPS 12. Additionally, the flexible circuit may be continuous and/or may include one or more holes (the holes may include at least a portion of the flexible circuit 14 in some examples).

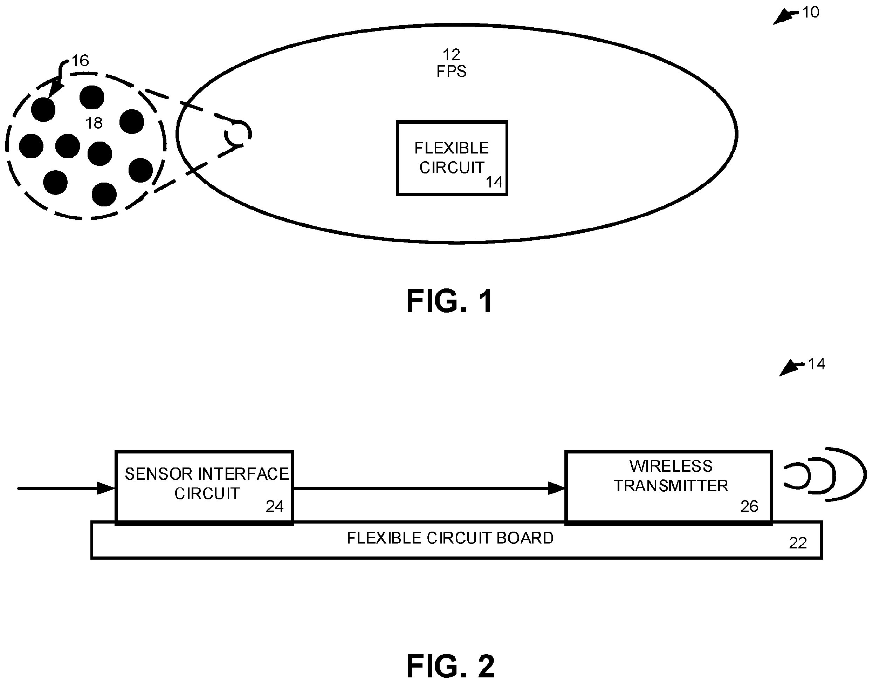

The FPS 12 of the present invention is made of a piezoresistive elastomer composite (shown in the blown out portion of FIG. 1). The piezoresistive elastomer composite includes a number of conductive particles 16 (e.g., micron or sub-micron sized, like nanoparticles) dispersed within the elastomer 18. As an example, the elastomer can be a biocompatible material that is very compliant, such as PDMS (however, other elastomers can be used). The conductive particles 16 can include one or more materials, such as a material including an activated carbon-containing material, of any geometric configuration. Generally the carbon-containing material can have a low toxicity and provide fewer impurities. Different carbon materials can impart different properties relating to flexibility and sensitivity of the FPS 12. Examples include, but are not limited to, carbon nanoparticles, carbon black, graphene flakes, or the like.

As one example, the conductive particles 16 can include a matrix of multi-walled carbon nanotubes (MWCNT) dispersed in PDMS. In an example, the FPS 12 can be configured to exhibit an isotropic compliance along a given direction with respect to a direction transverse to the given direction. For instance, the FPS 12 can be strain-sensitive along one direction and exhibit compliance up to about 20% (or greater) in another (strain sensitive) direction such that the FPS 12 is elastically deformable along the direction, but is non-deformable in the other transverse direction. In some instances, the FPS 12 can include the conductive particles 16 (e.g., micron or sub-micron sized, like nanoparticles) dispersed throughout the elastomer 18 to impart a piezoresistive effect and then a conductive polymer placed in one or more layers within a non-conductive polymer to form the FPS 12.

The FPS 12 can be connected to one or more pads of the flexible circuit 14. The flexible circuit 14, as shown in FIGS. 2-5 can include a flexible circuit board 22 with elements printed thereon (but not illustrated as such - the elements are illustrated above the flexible circuit board 22 in FIGS. 2-5 for ease of illustration).

As shown in FIG. 2, at least a sensor interface circuit 24 (referred to interchangeably as sensor interface 24) and a wireless transmitter 26 is on the flexible circuit board 22. The sensor interface 24 can measure electrical property changes in the FPS 12 related to displacement of the FPS. The displacement of the FPS can be related to a pressure of and/or within the measurement target. For example, the displacement of the FPS can be related to a blood pressure and/or a heart rate detected in a vessel or synthetic vascular graft. A vessel can be a vein or an artery.

In some instances, the sensor interface 24 can include a bridge circuit that is on the flexible circuit board 22 to measure the displacement of the FPS caused by the pressure of and/or within the measurement target. In some instances, the sensor interface 24 can perform additional data processing tasks on the data received from the FPS 12 and/or the data determined by another sensor mounted on the circuit board, e.g. a pressure sensor. The sensor interface 26 can send the processed data to the wireless transmitter 26.

The wireless transmitter 26 can be configured for data transmission (and in some instances, reception). In other words, the wireless transmitter 26 can always be configured to transmit data to an external device, but in some instances, can be configured to receive instructions from the external device. For example, the wireless transmitter 26 can be configured for short range transmission (or reception). For example, the wireless transmitter 26 may be implemented as an inductive communications link or according to another wireless technology, such as 802.11x Wi-Fi, Bluetooth, ZigBee, cellular or the like that can communicate the data to external device. For example, the external device may be smart phone, server or other wireless receiver.

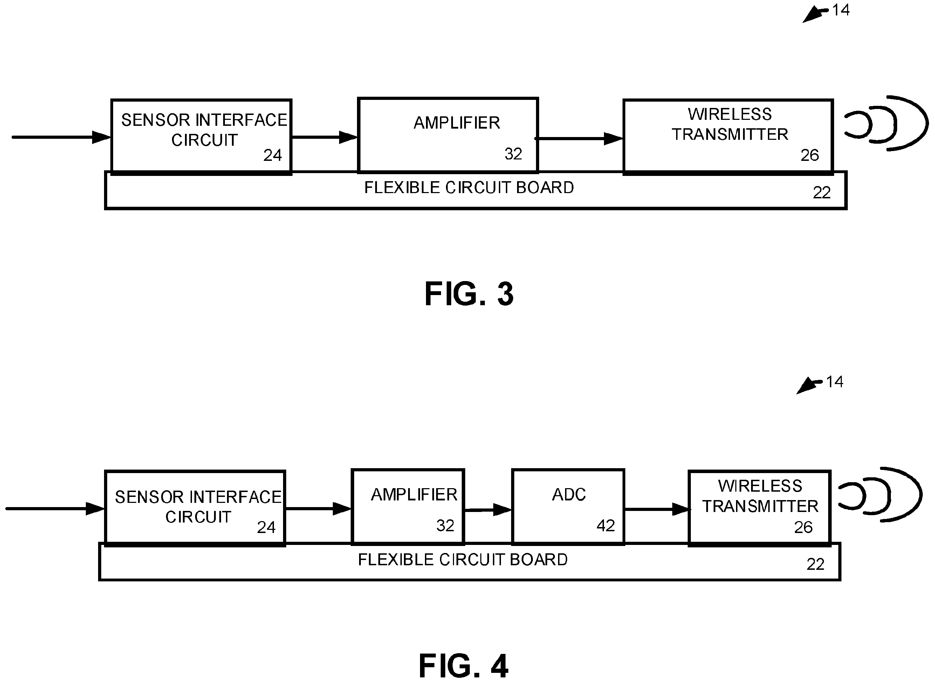

The circuit elements of FIGS. 3-5 can be embodied in the external device, alternatively. But one or more of the configurations of the flexible circuit 12 may be possible. As shown in FIG. 3, the flexible circuit 14 of the present invention includes an amplifier 32 on the flexible circuit board. As illustrated in FIGS. 3-5, the amplifier 32 receives data from the sensor interface 24 and processes the data before being sent to the wireless transmitter 26. The amplifier 32 is configured to optimize bandwidth and/or gain of the data so that the data fits within a full-scale range (e.g., 2 Volts, but the range can be wider or more narrow). For example, the gain of the amplifier can be selected and/or the filter characteristics can be selected based on one or more simulations (e.g., using electrical simulation software).

As shown in FIG. 4, the amplified data can be converted from analog to digital (e.g., by ADC 42) before being sent by the wireless transmitter 26. For example, the electrical resistance may be measured in response to application of an AC signal (e.g., pulses). As mentioned, the electrical resistance of the conductive layer varies as a function of its length, corresponding to circumferential strain of the graft 54 and sensor apparatus. (e.g., operating as a strain gauge). The electrical resistance of the FPS 12 may be determined to provide measurement data that is stored in memory (not shown). The ADC 42 may convert the analog electrical resistance into digital values that can be transmitted.

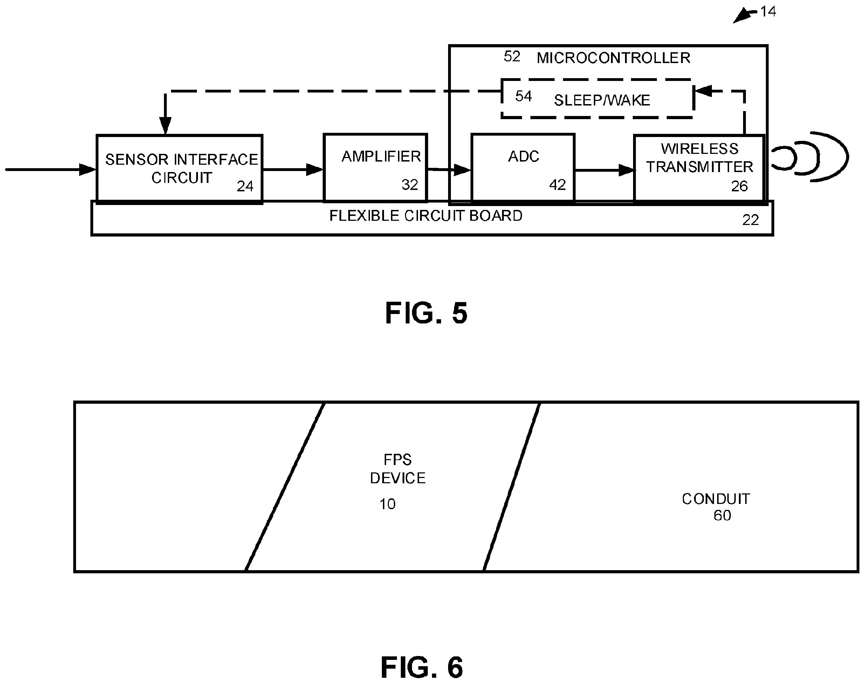

As shown in FIG. 5, the flexible circuit 14 can include a microcontroller 52. The microcontroller 52 can include the ADC 42 and the wireless transmitter 44 (the ADC 42 can be considered to be part of the wireless transmitter 44) integrated therewithin. In some instances, the amplifier 32 may be integrated within the microcontroller 52, but in other instances, the amplifier 32 can be separate from the microcontroller 52.

In some instances, the wireless transmitter can be bi-directional and receive instructions from the external device. As an example, the instructions can be related to a sleep/wake characteristic of the FPS device 10. The sleep/wake signal 54 can be received and the sensor interface 24 and microcontroller 52 can begin recording the pressure detected by the FPS 12 (until receiving a stop or sleep signal). In some instances, the sleep/wake signal 54 can conserve battery of the FPS device 10.

Referring now to FIG. 6, as illustrated, the FPS device 10 can be wrapped around the conduit 60 at a measurement target. It should be noted that the FPS device 10 can be thin when compared to the width of the conduit 60 (e.g., the width of the conduit 60 is >> than a thickness of the FPS device 10). The conduit 60 can be any vessel, such as an artery or a vein, a synthetic graft, an autograft, or the like.

The following description refers to the conduit 60 being a synthetic graft. It should be noted that the FPS device 10 is flexible such that the FPS device 10 can be applied separately from the graft and allows use on native vessels because the FPS device 10 can be applied operatively without crushing the vessel or during graft implantation procedures to remain implanted. To realize such capabilities, the FPS device 10 includes piezoresistive elastomer composites as the FPS 12 transduction material (e.g., a composite of PDMS and conductive nanoparticles can provide a robust piezoresistive strain response). The flexible circuit 14 can provide for wireless transmission on a flexible circuit board 22. The flexible circuit 14 is bonded to the conductive PDMS composite.

In FIG. 6, the example of the FPS device 10 can be placed around the measurement target on an external portion of a conduit 60, a graft or a vessel (artery or vein). For example, the graft can be a hemodialysis access graft or other vascular graft. In this example, the end of the graft may be anastomosed to tissue to provide for flow of blood through the graft. In other examples, one or more FPS sensors 10, as described herein, may be applied to other surfaces (e.g., biological tissue or synthetic) for sensing deformation of such surface in one or more directions.

For example, the graft includes a sidewall that extends cylindrically between two ends. In this example, the FPS device 10 can be mounted around the sidewall as to circumscribe (partially or wholly) the graft. The FPS device 10 may be mounted to the graft such that a radially inner surface (e.g., bottom layer) of the FPS device 10 approximates the outer diameter of the sidewall. The FPS 12 can have a compliance that is commensurate with or greater than the compliance of the sidewall of the graft or the vessel. In this way, deformation of the sidewall, such as occurs in the pulsation of blood flow therethrough, results in corresponding deformation of the FPS 12.

As another example, the circumference of the FPS device 10 that engages the graft sidewall corresponds to a length of the apparatus that can be wrapped around or otherwise mounted to the sidewall (e.g., by sutures, an adhesive or the attachment mechanism). Since the FPS device 10 is fixed externally to the graft, the sensor apparatus can monitor graft motion based on the electrical resistance of the conductive layer, which changes based on deformation. As mentioned, the substrate and conductive layers may be anisotropically compliant to enable radial or circumferential deformation but prevent deformation in the axial dimension (along its width). As a result, the deformation causes a change in resistance that correlates to flow rate and/pressure through the graft. As a result, the FPS device 10 can enable detection of graft dysfunction without adversely affecting blood flow through the graft. In other examples, one or more FPS devices 10 can be attached to the sidewall of other tissue to monitor other tissue function. A compliant covering of a compliant biocompatible material further may be applied over the sensor apparatus and a portion of the adjacent sidewall.

Graft wall motion can be monitored based on the data measured by the FPS device 10. In response to detecting an increase or a decrease in graft wall motion above or below an established threshold, an occurrence of a dysfunction may be determined, such as an occlusion, stenosis or other physiological condition. An alert can be generated (e.g., by the external device) in response to determining the dysfunction. The alert may be communicated to the patient and/or one or more caregivers. In this way, additional testing may be performed to determine if an intervention may be required to avoid graft failure and extend graft patency.

The following description refers to the conduit 60 being any vessel, such as an artery, a vein, or an autograft, as described herein. The FPS device 10 can be applied operatively to the vessel without crushing the vessel, or otherwise impeding blood or fluid flow, by wrapping the FPS at least partially around the vessel. If the FPS device 10 is wrapped all the way around the vessel, then the two ends of the FPS device can be brought into apposition and attaching them together (e.g., threading sutures through holes on one or both ends of the FPS device, an adhesive to connect the two ends, a mechanical attachment, including a button, a ratchet, other types of mechanisms, or the like). If the FPS device 10 is wrapped only partially around the vessel, then the device may be attached to the vessel with any known means (adhesive, operative, mechanical, etc.). It should be noted that the FPS device 10 that is placed on a vessel has a commensurate or greater compliance than the vessel it is placed on so that the FPS device 10 can wrap around the vessel without causing constriction. An FPS device 10 that is wrapped around a vessel is about 2 times, 4 times, 6 times, 8 times, or 10 times or more flexible than an FPS device 10 that is placed on a synthetic graft. To realize such capabilities, the FPS device 10 includes piezoresistive elastomer composites as the FPS 12 transduction material (e.g., a composite of PDMS and conductive nanoparticles can provide a robust piezoresistive strain response). The flexible circuit 14 can provide for wireless transmission on a flexible circuit board 22. The flexible circuit 14 can be bonded to the conductive PDMS composite.

In FIG. 6, the example of the FPS device 10 can be placed around the conduit 60. In other examples, one or more FPS devices 10 can be wrapped around other native vessels or different portions of the same native vessel for sensing in more than one location. The FPS device 10 can measure the diastolic-systolic blood pressure waveform continuously with an accuracy within 3 mmHg for tens of thousands of cycles for, for example, neurostimulation feedback, heart/heart implant monitoring, blood pressure monitoring for diagnosis or treatment monitoring, etc. The FPS device 10 measures the vessel wall tension through the entire pressure waveform so blood pressure, flow phases, and heart rate can be measured simultaneously without contacting blood or interfering with a patient's normal activities.

Similar to the graft example discussed above, the FPS device 10 can circumscribe (partially or wholly) the vessel with a radially inner surface (e.g., bottom layer) of the FPS device approximating the outer diameter of a sidewall of the vessel. Because the FPS device 10, and FPS 12, has a compliance that is commensurate or greater than the compliance of the vessel it will not crush the vessel and can correspondingly deform with the sidewall based on the blood flow through the vessel. The piezoresistive nature of the FPS device 10 is important for more accurate measurements of blood pressure and/or heart rate than capacitive sensors can manage because the small capacitance changes created by the pulsatile flow of blood in an vessel past a capacitive sensor are difficult to near impossible to measure accurately due to the parasitic capacitance of the lead itself.

The FPS device 10 and the FPS 12 can be used for many applications, including monitoring hypertension, blood pressure, and other anatomical pressures. The FPS device 10 can be used as a standalone sensor for monitoring blood pressure, as an example. As another example, the FPS device 10 can be used to monitor pressure after endovascular surgery, transplant, hemodialysis vascular access, or the like. As a further example, the FPS device 10 can be used as a diagnostic. As an example, the FPS 12 can be used diagnostically as a long term Holter monitor replacement for patients with difficult to diagnose heart ailments, such as an arrhythmia or other electrical abnormality. The FPS device 10, as another example, can measure aspects of pulse pressure waves, such that physiologic activity can be indirectly gauged from the sensor.

Additionally, as a further example, the FPS device 10 can have GI applications, like measuring intestinal peristaltic pressure and may be applicable in devices that limit gastric emptying or as part of a device that is intended to go around the lower esophageal sphincter to limit gastro-esophageal reflux.

### IV. Method

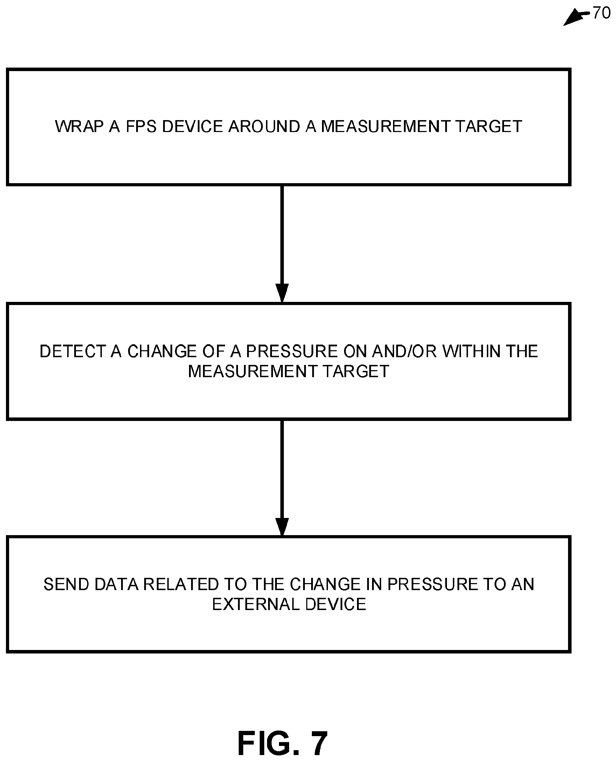

Another aspect of the present disclosure can include an example method 70 (shown in FIG. 7) for using a flexible pulsation sensor (FPS) device (e.g., the FPS device 10 shown in FIG. 1). The method 70 is illustrated as a process flow diagram with flowchart illustrations that can be implemented by one or more components of the FPS device 10, as shown in FIG. 1. For purposes of simplicity, the method 70 is shown and described as being executed serially; however, it is to be understood and appreciated that the present disclosure is not limited by the illustrated order as some steps could occur in different orders and/or concurrently with other steps shown and described herein. Moreover, not all illustrated aspects may be required to implement the method 70.

At step 72, a FPS device can be wrapped around a measurement target (e.g., as shown, for example, in FIG. 6; for example, the measurement target can be a conduit, like a synthetic vascular graft or a native vessel). The FPS device can include a FPS (e.g., made of a piezoresistive elastomer composite) configured to wrap around the measurement target and its displacement caused by the pressure of and/or within the measurement target; and a flexible circuit board (e.g., shown in FIGS. 2-5). As an example, the flexible circuit can include at least a pressure sensor on the flexible circuit board configured to collect the data related to displacement of the FPS related to a pressure of and/or within the measurement target; and a wireless transmitter on the flexible circuit board configured to transmit the data related to the pressure of and/or within the measurement target wirelessly to the external device. The measurement target can be, for example, a conduit, such as a native vessel (artery, vein), a synthetic graft, or the like. Other cylindrical or tubular structures may be similarly instrumented to measure strain or movement, for example, bones, intestines, or synthetic musculoskeletal implants, or the like.

At 74, a change of a pressure on and/or within the measurement target can be detected (e.g., by the FPS device 10). As noted, the change of the pressure (or the pulsation) can be reflected in a displacement of the FPS. At 76, data related to the change in the pressure can be sent to an external device (e.g., by a wireless transmitter 26 of the FPS device 10).

### V. Experimental

### First Experiment

The following experiment shows that a flexible pulsation sensor (FPS) device can be integrated with a flexible wireless transmitter for real-time data readout to form a "FPS device". A conductive polymer sensing layer was attached to a flexible circuit board and then encapsulated by polydimethylsiloxane (PDMS) for biocompatibility. Due to the FPS' outstanding flexibility in comparison to natural arteries, veins, and synthetic vascular grafts, the FPS device can be wrapped around target conduits to monitor blood pressure for short-term surgical and long-term implantation purposes. In this experiment, the power spectrum of the FPS data was analyzed to determine the ideal bandwidth of the wireless FPS device to preserve heart rate and hemodynamic waveforms while rejecting noise. The following experimental results are shown for the purpose of illustration only and are not intended to limit the scope of the appended claims.

The power spectrum of previously collected FPS data was analyzed to determine the ideal bandwidth of the wireless FPS device to preserve heart rate and hemodynamic waveforms while rejecting noise. By analyzing the power spectrum density of data collected previously, signals below 2 Hz contained more than 95% of the power (FIG. 8, elements a and b). This suggested that the hemodynamic pressure waveform was generally constrained to a bandwidth about twice the heart rate. Since the heart rate of adults normally ranges from 60 to 180 beats per minute based on activity level, the bandwidth of the FPS amplifier interface was designed to be 0.2-10 Hz. This effectively filters high frequency noise and low-frequency baseline drift typical of piezoresistive composite materials, while optimally preserving the hemodynamic waveform from the FPS (FIG. 8, element c).

The FPS interface amplifier (FIG. 9) was designed to have optimized bandwidth and gain to transform the FPS resistive response to a full-scale range of 2 V for data conversion. Component selection to tune the 3-dB frequency response and front-end amplifier gain was performed through SPICE simulation (Table 1).

**TABLE 1: MEASURED PERFORMANCE OF SENSOR PROTOTYPE**

| **Name** | **Value** | **Specification** | **Measured Value** |
|---|---|---|---|
| R_{B} | 100 kΩ | Bandwidth | 0.2 - 30 Hz |
| R₁ | 100 kΩ | Dynamic Range | 8 bits |
| C₁ | 4.7 µF | Sample Rate | 100 Hz |
| R₂ | 2.8 MΩ | Decimated Sample Transmission Rate | 30 Hz |
| C₂ | 5.6 nF | Current Draw | 86 µA |
| | | Transmit Range | 30 cm |

Wireless readout from the sensor was enabled using a low- power microcontroller with integrated analog-to-digital converter (ADC) and data signal modulator peripheral (DSM). The FPS resistance change was measured by a bridge circuit, amplified and filtered, and digitized by the ADC to 8-bit resolution at 100 samples/s. A digital decimation filter reduced the data rate to 33 Hz. Data were then transmitted every 100 ms to save power; each transmission included 3 sequential samples within a data packet. Transmission used Manchester-encoded on-off-keying of a 4 MHz carrier using the DSM. A resonant LC circuit with a matching capacitor was used to enable short-range inductive communication to an external data receiver.

The microcontroller software was designed to transmit data continuously for 15 minutes before entering a low-power sleep state. The wireless FPS was activated from sleep by applying a large RF pulse which was received by the inductive antenna to trigger a wake-from-sleep interrupt. This activation system is needed to save power in an implanted application. However, for demonstration in wired leads were soldered to the device instead of a battery.

The fabrication process of the conductive PDMS sensing layer was modified in order to connect the FPS to the flexible circuit board. First, a pure PDMS substrate (Ecoflex 00-10) was fabricated on a flexible transparency film. Complete degassing was needed in this step to ensure there were no air bubbles in the substrate. Next, a rectangular hole with the same size of the flexible circuit board was cut into the substrate. The flexible circuit board was designed with circuitry on the front side and connection pads for the FPS on the back; electroless nickel followed by immersion gold plating was used to provide a gold contact layer to the conductive sensor composite. The flexible circuit board was placed in the rectangular hole with the side of FPS contact pads facing up. A stencil was then placed and aligned on the PDMS substrate and conductive PDMS paste was cast over the stencil. After removing the stencil and curing for 30 minutes at 80°C, another layer of pure PDMS was applied and cured over the FPS and flexible circuit board. The sandwich structure was carefully peeled off, flipped and placed on a glass slide. At this time, the conductive sensing layer was reliably connected to the flexible circuit board and the other side of the board was exposed for the soldering of electronic components. Solder paste (Indium 8.9E) was then applied under microscope using a dispensing tool (Nordson EFD X100), following by component placement and reflow soldering (Puhui T-962). Two stainless steel wires were soldered to the board for connections to a power supply (BK Precision 1550). Then, small drops of medical grade epoxy (Loctite EA M-121HP) were applied to the soldering joints to mechanically protect the soldering and to act as moisture barriers. Finally, another layer of pure PDMS was cast over the flexible board as the encapsulation. Pictures of the prototype device in different fabrication stages are shown in FIG. 10.

After fabrication, the FPS was connected to a 3 V power supply and wrapped around a 6-mm silicone tube simulating a peripheral blood vessel for testing (FIG. 11). The system used a peristaltic roller pump (Cole Parmer Masterflex L/S 07522) and a variable-voltage diaphragm pump (Shurflo 4008) to produce arterial pressure waveforms. Blood-mimicking fluid simulated shear-thinning properties of blood flow. The silicone tube was connected between the high and low pressure systems of the phantom to simulate an arteriovenous vascular access graft. Low-resistance pressure sensors (Pendotech PREPS-N-50) monitored the graft inlet (arterial) and outflow (venous) pressures and an electromagnetic flow meter (Omega FMG90) measured pulsatile and average flow rate. An antenna was placed about 20 cm from the FPS for data reception using a custom 4-MHz receiver system. The wireless radio was connected to a laptop for saving the data for further analysis

FIG. 12 shows the wireless radio used for data recording and the receiving antenna held 30 cm from the wireless FPS prototype. The wireless radio consisted of a custom 4-MHz on- off-keying receiver and Teensy 3.6 USB development board running data demodulating and recording software. Received data were saved to a microSD card and transmitted to a PC over USB serial port for display in a custom LABVIEW program. FPS data points sampled at 30 Hz were reconstructed in MATLAB and filtered using a 5^{th}-order, length 9 Savitzky-Golay to filter out single-bit ADC errors.

The prototype was tested under different pressure settings by changing the driven voltage of the diaphragm pump from 4- 10 V, producing flow rates of 200-700 mL/minute and systolic-diastolic pressures of 70-190 mmHg in the vascular phantom. FIG. 13 shows the pressure data and wirelessly transmitted voltage signal. There was good agreement between the Vout data and the pulsatile blood pressure waveform. The peak to peak voltage of Vout versus peak to peak and RMS pressure at different pump driven voltage varied linearly (FIG. 14). In this example test the wireless FPS measured blood pressure with an accuracy of ±3.3 mmHg, or 5.2% error. The results indicate that the peak-to-peak FPS voltage responds linearly to RMS blood pressure and systolic- diastolic pressure.

### Second Experiment

As demonstrated in the Second Experiment described below, a flexible pulsation sensor (FPS) can accurately measure changes in blood pressure.

### Methods

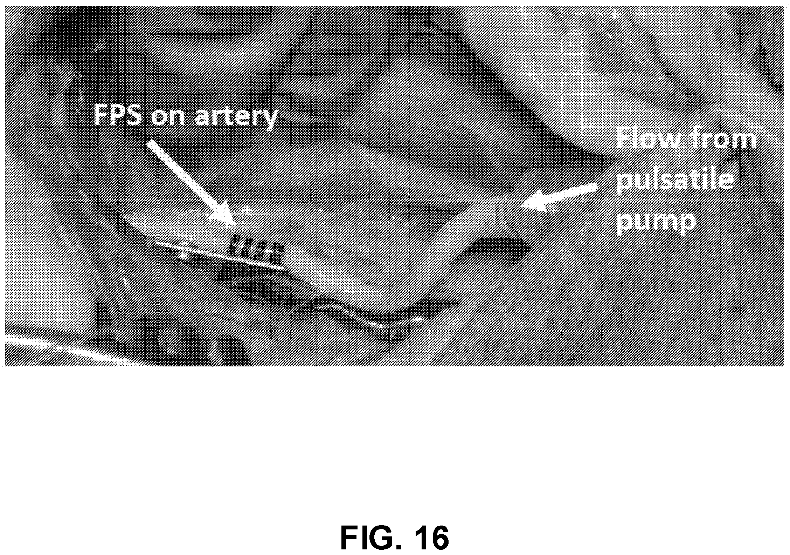

A cadaveric adult Yucatan minipig weighing approximately 30 kg was used in this experiment. The neck of the minipig was dissected to expose the carotid arteries and the carotid sinus nerve (shown in FIG. 15). The FPS was wrapped around the internal carotid artery and secured with a bulldog clamp. After the FPS was secured with the clamp, the artery was connected to a pulsatile pump through silicon tubing and pressurized (the pulsatile pump had at least 3V power to give a pressure of approximately 125/75 mmHg) (FIG. 16). Data recorded by the FPS was not calibrated. After pressurization, if distension of the internal carotid artery was limited, the FPS was determined to be wrapped too tightly around the artery.

The pulsatile pump was connected to the artery with a silicon tube, which was connected to the artery with a barbed coupler up-stream of the FPS's location. The silicon tubing was approximately 2.2 m long, with a pressure reference sensor (to measure reference pressure) located approximately 20 cm up-stream of the barbed coupler.

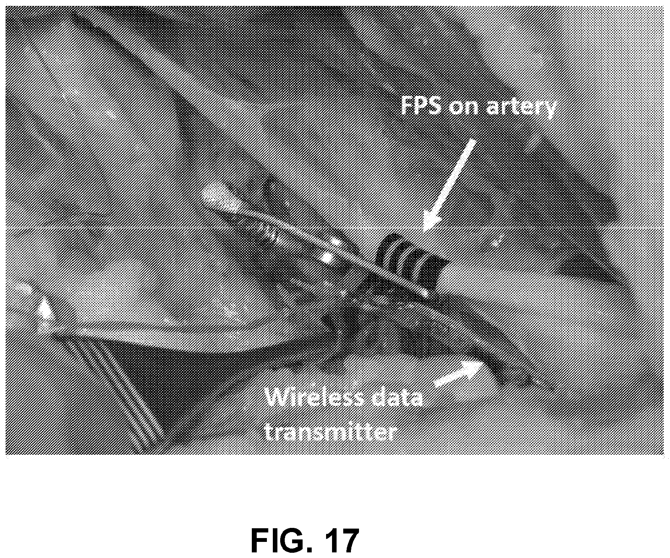

A wireless data transmitter was attached to the FPS and positioned near the artery (FIG. 17). The data was collected wirelessly from the FPS and transmitted through the wireless data transmitter to a receiving antenna (FIG. 18, element a showing the receiving antenna and element b showing the data transmission from the FPS through the wireless data transmitter). The data included arterial pressures measured using a blood pressure transducer in the FPS. The collected data was post-processed using a computer in communication with the receiving antenna.

### Results

Example data was recorded that showed correlations in flow- and heart-rate measures from the reference pressures sensor and the FPS on the internal carotid artery, graphs include filtered and not filtered data (FIGS. 19 and 20). As shown in FIG. 21, and in further detail in FIG. 22, as the pump pressure was increased there was stagnation in the FPS signal, which was likely caused by over--pressurization. The plotted data were filtered and the graphs do not show the DC shift in pressure.

There was a high correlation between data collected from the pressure reference sensor on the tubing and the data collected from the FPS. Because the FPS data was not calibrated it was expressed in arbitrary units (AU). A modeled heart rate could be clearly distinguished by the FPS. Additionally, lower FPS sensitivity would be due to the FPS being too tightly wrapped around the artery.

From the above description, those skilled in the art will perceive improvements, changes and modifications. Such improvements, changes and modifications are within the skill of one in the art and are intended to be covered by the appended claims.

## Claims

1. An apparatus (10) comprising:
a flexible pulsation sensor (FPS) (12) comprising a piezoresistive elastomer composite (18) having conductive particles dispersed therein, the FPS (12) being configured to wrap around a vessel or graft without impeding fluid flow through the vessel or the graft and measure displacement of the piezoresistive elastomer composite (18) due to pulsatile fluid flow through the vessel or graft,
wherein the displacement of the piezoresistive elastomer composite (18) is measured as electrical resistance data that is a function of the length of the FPS (12), which corresponds to a circumferential strain of the vessel or the graft, and
wherein the displacement of the piezoresistive elastomer composite (18) is related to a pressure of and/or within the vessel or the graft and configured to be measured through an entire diastolic-systolic blood pressure waveform; and
a flexible circuit board (14, 22) bonded to the FPS (12) and electrically connected to the FPS (12), the flexible circuit board (14, 22) comprising:
a sensor interface (24) on the flexible circuit board (22) configured to collect the electrical resistance data related to the displacement of the piezoresistive elastomer composite (18) of the FPS (12);
a wireless transmitter (26) on the flexible circuit board (22) configured to transmit the data related to the displacement wirelessly to an external device; and
an amplifier (32) on the flexible circuit board (22) configured to interface with the sensor interface (24) and the wireless transmitter (26) to bandpass filter the data and amplify the data to fit within a full scale voltage range before the wireless transmitter (26) transmits the data to the external device.

2. The apparatus of claim 1, wherein the elastomer comprises polydimethylsiloxane (PDMS).

3. The apparatus of claim 1 or 2, wherein the conductive particles are nanoparticles.

4. The apparatus of any preceding claim, wherein the wireless transmitter (26) comprises an analog to digital converter (42).

5. The apparatus of claim 4, wherein the wireless transmitter (26) is a microcontroller (52) with the analog to digital converter (42) integrated therewithin.

6. The apparatus of claim 5, wherein the microcontroller (52) is configured to wake from sleep upon receiving a signal (54) from the external device.

7. The apparatus of any preceding claim, wherein the sensor interface (24) comprises a bridge circuit on the flexible circuit board (22).

8. The apparatus of claim 1, wherein the graft comprises an autograft, an allograft, a xenograft, or a synthetic graft.

9. The apparatus of any preceding claim, wherein the apparatus is configured to measure at least one value indicative of a blood pressure and/or a heart rate of a patient.

10. The apparatus of any preceding claim, wherein the flexible circuit board (14) is bonded to the piezoresistive elastomer composite (18) of the FPS (12).

11. The apparatus of claim 5, wherein the microcontroller (52) is further configured to determine an occurrence of a dysfunction based on data collected over a time and wherein the external device in communication with the microcontroller is configured to generate an alert to be communicated to a patient and/or one or more caregivers in response to determining the occurrence of the dysfunction.

12. The apparatus of any preceding claim, wherein the piezoresistive elastomer composite (18) of the FPS (12) is elastically deformable in a first direction and non-deformable in a second direction transverse to the first direction.

13. The apparatus of any preceding claim, wherein the piezoresistive elastomer composite (18) is a layer of the FPS (12) and the FPS (12) further comprises at least one conductive polymer layer overlaying the piezoresistive elastomer composite.

## Patentansprüche

1. Einrichtung (10), umfassend:
einen flexiblen Pulsationssensor (FPS) (12), der einen piezoresistiven Elastomer-Verbundwerkstoff (18) umfasst, der darin dispergierte leitfähige Partikel aufweist, wobei der FPS (12) konfiguriert ist, ein Gefäß oder Transplantat zu umhüllen, ohne den Flüssigkeitsfluss durch das Gefäß oder das Transplantat zu behindern, und die Verschiebung des piezoresistiven Elastomer-Verbundwerkstoffs (18) aufgrund des pulsierenden Flüssigkeitsflusses durch das Gefäß oder Transplantat zu messen,
wobei die Verschiebung des piezoresistiven Elastomer-Verbundwerkstoffs (18) als elektrische Widerstandsdaten gemessen wird, die eine Funktion der Länge des FPS (12) sind, was einer Umfangsdehnung des Gefäßes oder des Transplantats entspricht, und
wobei sich die Verschiebung des piezoresistiven Elastomer-Verbundwerkstoffs (18) auf einen Druck von und/oder innerhalb des Gefäßes oder des Transplantats bezieht und konfiguriert ist, über eine gesamte diastolisch-systolische Blutdruckkurve gemessen zu werden; und
eine flexible Leiterplatte (14, 22), die an das FPS (12) gebunden und elektrisch mit dem FPS (12) verbunden ist, wobei die flexible Leiterplatte (14, 22) umfasst:
eine Sensorschnittstelle (24) auf der flexiblen Leiterplatte (22), die konfiguriert ist, die elektrischen Widerstandsdaten in Bezug auf die Verschiebung des piezoresistiven Elastomer-Verbundwerkstoffs (18) des FPS (12) zu sammeln;
einen drahtlosen Sender (26) auf der flexiblen Leiterplatte (22), der konfiguriert ist, die Daten in Bezug auf die Verschiebung drahtlos an eine externe Vorrichtung zu senden; und
einen Verstärker (32) auf der flexiblen Leiterplatte (22), der konfiguriert ist, mit der Sensorschnittstelle (24) und dem drahtlosen Sender (26) zu vernetzen, um die Daten bandpasszufiltern und die Daten zu verstärken, damit sie in einen Spannungsbereich mit vollem Skalenwert passen, bevor der drahtlose Sender (26) die Daten an die externe Vorrichtung sendet.

2. Einrichtung nach Anspruch 1, wobei das Elastomer Polydimethylsiloxan (PDMS) umfasst.

3. Einrichtung nach Anspruch 1 oder 2, wobei es sich bei den leitfähigen Partikeln um Nanopartikel handelt.

4. Einrichtung nach einem vorstehenden Anspruch, wobei der drahtlose Sender (26) einen Analog-Digital-Wandler (42) umfasst.

5. Einrichtung nach Anspruch 4, wobei der drahtlose Sender (26) ein Mikrocontroller (52) mit dem darin integrierten Analog-Digital-Wandler (42) ist.

6. Einrichtung nach Anspruch 5, wobei der Mikrocontroller (52) konfiguriert ist, beim Empfang eines Signals (54) von der externen Vorrichtung aus dem Schlafmodus aufzuwachen.

7. Einrichtung nach einem vorstehenden Anspruch, wobei die Sensorschnittstelle (24) eine Brückenschaltung auf der flexiblen Leiterplatte (22) umfasst.

8. Einrichtung nach Anspruch 1, wobei das Transplantat ein Autotransplantat, ein Allotransplantat, ein Xenotransplantat oder ein synthetisches Transplantat umfasst.

9. Einrichtung nach einem vorstehenden Anspruch, wobei die Einrichtung konfiguriert ist, mindestens einen Wert zu messen, der einen Blutdruck und/oder eine Herzfrequenz eines Patienten angibt.

10. Einrichtung nach einem vorstehenden Anspruch, wobei die flexible Leiterplatte (14) an den piezoresistiven Elastomer-Verbundwerkstoff (18) des FPS (12) gebunden ist.

11. Einrichtung nach Anspruch 5, wobei der Mikrocontroller (52) weiter konfiguriert ist, ein Auftreten einer Funktionsstörung anhand von über einen bestimmten Zeitraum gesammelten Daten zu bestimmen, und wobei die externe Vorrichtung, die mit dem Mikrocontroller in Kommunikation steht, konfiguriert ist, als Reaktion auf Bestimmen des Auftretens der Funktionsstörung einen Alarm zu erzeugen, der an einen Patienten und/oder einen oder mehrere Pflegekräfte kommuniziert wird.

12. Einrichtung nach einem vorstehenden Anspruch, wobei der piezoresistive Elastomer-Verbundwerkstoff (18) des FPS (12) in einer ersten Richtung elastisch verformbar und in einer zweiten Richtung, die quer zur ersten Richtung liegt, nicht verformbar ist.

13. Einrichtung nach einem vorstehenden Anspruch, wobei der piezoresistive Elastomer-Verbundwerkstoff (18) eine Schicht des FPS (12) ist und das FPS (12) weiter mindestens eine leitfähige Polymerschicht umfasst, die den piezoresistiven Elastomer-Verbundwerkstoff überlagert.

## Revendications

1. Appareil (10) comprenant :
un capteur de pulsation flexible (FPS) (12) comprenant un composite élastomère piézorésistif (18) présentant des particules conductrices dispersées dans celui-ci, le FPS (12) étant configuré pour s'enrouler autour d'un vaisseau ou d'une greffe sans entraver un écoulement de fluide à travers le vaisseau ou la greffe et mesurer le déplacement du composite élastomère piézorésistif (18) dû à l'écoulement pulsatile de fluide à travers le vaisseau ou la greffe,
dans lequel le déplacement du composite élastomère piézorésistif (18) est mesuré sous forme de données de résistance électrique qui sont fonction de la longueur du FPS (12), ce qui correspond à une déformation circonférentielle du vaisseau ou de la greffe, et
dans lequel le déplacement du composite élastomère piézorésistif (18) est lié à une pression et/ou à l'intérieur du vaisseau ou de la greffe et configuré pour être mesuré sur toute une forme d'onde de pression artérielle diastolique-systolique ; et
une carte de circuit imprimé flexible (14, 22) collée au FPS (12) et reliée électriquement au FPS (12), la carte de circuit imprimé flexible (14, 22) comprenant :
une interface de capteur (24) sur la carte de circuit imprimé flexible (22) configurée pour collecter les données de résistance électrique liées au déplacement du composite élastomère piézorésistif (18) du FPS (12) ;
un émetteur sans fil (26) sur la carte de circuit imprimé flexible (22) configuré pour transmettre sans fil les données relatives au déplacement à un dispositif externe ; et
un amplificateur (32) sur la carte de circuit imprimé flexible (22) configuré pour s'interfacer avec l'interface de capteur (24) et l'émetteur sans fil (26) pour filtrer passe-bande les données et les amplifier afin de les adapter à une plage de tension pleine échelle avant que l'émetteur sans fil (26) ne transmette les données au dispositif externe.

2. Appareil selon la revendication 1, dans lequel l'élastomère comprend du polydiméthylsiloxane (PDMS).

3. Appareil selon la revendication 1 ou 2, dans lequel les particules conductrices sont des nanoparticules.

4. Appareil selon une quelconque revendication précédente, dans lequel l'émetteur sans fil (26) comprend un convertisseur analogique-numérique (42).

5. Appareil selon la revendication 4, dans lequel l'émetteur sans fil (26) est un microcontrôleur (52) auquel le convertisseur analogique-numérique (42) est intégré.

6. Appareil selon la revendication 5, dans lequel le microcontrôleur (52) est configuré pour se réveiller à la réception d'un signal (54) provenant du dispositif externe.

7. Appareil selon une quelconque revendication précédente, dans lequel l'interface de capteur (24) comprend un circuit de pont sur la carte de circuit imprimé flexible (22).

8. Appareil selon la revendication 1, dans lequel la greffe comprend une autogreffe, une allogreffe, une xénogreffe ou une greffe synthétique.

9. Appareil selon une quelconque revendication précédente, dans lequel l'appareil est configuré pour mesurer au moins une valeur indiquant une pression artérielle et/ou un rythme cardiaque d'un patient.

10. Appareil selon une quelconque revendication précédente, dans lequel la carte de circuit imprimé flexible (14) est collée au composite élastomère piézorésistif (18) du FPS (12).

11. Appareil selon la revendication 5, dans lequel le microcontrôleur (52) est en outre configuré pour déterminer une survenance d'un dysfonctionnement sur la base de données collectées au fil du temps et dans lequel le dispositif externe en communication avec le microcontrôleur est configuré pour générer une alerte à communiquer à un patient et/ou à un ou plusieurs soignants en réponse à la détermination de la survenance du dysfonctionnement.

12. Appareil selon une quelconque revendication précédente, dans lequel le composite élastomère piézorésistif (18) du FPS (12) est élastiquement déformable dans une première direction et non déformable dans une seconde direction transversale à la première direction.

13. Appareil selon une quelconque revendication précédente, dans lequel le composite élastomère piézorésistif (18) est une couche du FPS (12) et le FPS (12) comprend en outre au moins une couche de polymère conducteur recouvrant le composite élastomère piézorésistif.
